(19) Europäisches Patentamt
European Patent Office
Office européen des brevets

(11) Numéro de publication: **0 250 325 B1**

(12) # FASCICULE DE BREVET EUROPEEN

(45) Date de publication de fascicule du brevet: **29.01.92**

(51) Int. Cl.⁵: **C07C 219/08**

(21) Numéro de dépôt: **87401373.3**

(22) Date de dépôt: **18.06.87**

(54) **Procédé de préparation de solution aqueuse de sels d'ammonium quaternaire insaturé.**

(30) Priorité: **20.06.86 US 876602**

(43) Date de publication de la demande:
**23.12.87 Bulletin 87/52**

(45) Mention de la délivrance du brevet:
**29.01.92 Bulletin 92/05**

(84) Etats contractants désignés:
**AT BE CH DE ES FR GB GR IT LI LU NL SE**

(56) Documents cités:
**GB-A- 1 538 265**

(73) Titulaire: **ATOCHEM**
**4 & 8, Cours Michelet La Défense 10**
**F-92800 Puteaux(FR)**

(72) Inventeur: **Hess, Raymond**
**21, rue Lepinseck**
**F-57600 Forbach(FR)**
Inventeur: **Lacroix, Christian**
**11, rue de l'Eglise**
**F-57600 Forbach(FR)**

(74) Mandataire: **Rieux, Michel et al**
**ATOCHEM Département Propriété Industriel-le 4, Cours Michelet - La Défense 10 - Cedex 42**
**F-92091 Paris-La Défense(FR)**

EP 0 250 325 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à un procédé de préparation de solution aqueuse de sels d'ammonium quaternaire insaturés (ci-après dénommés sels quaternaires) répondant à la formule (I) suivante :

$$H_2C = C(R_3) - C(O) - A - R_4 - \overset{\oplus}{N}(R_1)(R_2)(R), X^{\ominus}$$

dans laquelle :
- A est un atome d'oxygène ou un groupe NH,
- $R_3$ est un atome d'hydrogène ou un radical méthyle,
- $R_4$ est un radical alkyle, linéaire ou ramifié, de 2 à 4 atomes de carbone,
- $R_1$ et $R_2$, différents ou identiques, est un radical alkyle ou un radical aryle,
- R est un radical alkyle ou aryle,
- X est choisi parmi Cl, Br, I, $-CH_3-CO_3$ ou $-CH_3-SO_4$,

les sels quaternaires (I) étant obtenus en faisant réagir un monomère (méth)-acrylique (II) de formule :

$$H_2C = C(R_3) - C(O) - A - R_4 - N(R_1)(R_2)$$

avec un agent quaternisant (III) de formule RX.

On utilise des solutions aqueuses de sels quaternaires (I) pour préparer des polymères destinés à servir de floculants dans le traitement des eaux.

Les principaux problèmes qui se posent lors de la synthèse des sels quaternaires (I) sont, d'une part, la sensibilité à l'eau des monomères (II), et d'autre part, les risques de polymérisation du milieu réactionnel dans le réacteur et ceux des sels quaternaires (I) au stockage.

Dans le but de résoudre ces problèmes, de nombreuses synthèses des sels quaternaires (I), qui reprennent le principe décrit ci-dessus, ont été mises au point.

Une première catégorie de synthèses propose l'utilisation de solvants organiques tels que l'acétone (demande de brevet japonais N° 60/054343) ou des solvants cétoniques (demande de brevet allemand N° 3 244 274) qui permettent de limiter, voire d'éliminer les contacts eau-monomères (II) et donc de réduire les phénomènes d'hydrolyse de ces derniers. En fin d'opération, on récupère la phase aqueuse comprenant le sel quaternaire (I), par exemple par décantation. Cette phase aqueuse doit ensuite être purifiée afin d'éliminer les traces de solvants qui risquent d'une part de gêner la polymérisation du sel quaternaire (I), et, d'autre part, de compromettre les performances de ces polymères lors de leur utilisation notamment en tant que floculant.

Dans une seconde catégorie de synthèses, on effectue la quaternisation des monomères (II) en solution aqueuse uniquement. Ainsi, on a proposé un procédé selon lequel on fait réagir un monomère (II) à une concentration initiale dans l'eau d'au moins 80% en poids et pouvant atteindre 100% en poids (voir brevet GB 1 538 265). En effet, on constate qu'à de telles concentrations, les phénomènes d'hydrolyse des monomères (II) sont considérablement réduits. Toutefois, le procédé décrit dans le brevet GB 1 538 265 conduit à la formation, lors de la synthèse, de solutions sursaturées en sels quaternaires (I). Il y a donc précipitation de cristaux de sels et dépôts de cristaux sur les parois du réacteur, en particulier sur la surface de refroidissement prévue pour l'évacuation de la chaleur de réaction. Ces dépôts sont à l'origine de deux principales sortes d'ennuis. D'une part, comme indiqué ci-dessus, ils compromettent le refroidissement du mélange réactionnel. D'autre part, en dépassant le point de saturation, on obtient un milieu réactionnel possédant une viscosité élevée et une réduction considérable de la cinétique de réaction. A l'échelle du laboratoire, ces problèmes passent relativement inaperçu, par contre, à l'échelle industrielle, Ils se traduisent par un mauvais contrôle de la température de réaction avec des emballements de la température du réacteur, préjudiciables à la stabilité à la polymérisation du sel quaternaire (I) lors de son stockage.

Il est connu que la présence d'oxygène en quantité suffisante permet d'inhiber les processus de la polymérisation. Néanmoins, la mise en oeuvre au stade industriel de cette propriété est actuellement loin d'être satisfaisante. En utilisant les procédés connus de quaternisation, on est actuellement confronté à l'éventualité d'une polymérisation du sel quaternaire (I) lors de sa fabrication ou lors de son stockage. Pour cette raison, il est d'usage de préparer les solutions aqueuses de sels quaternaires (I) dans des réacteurs de taille modeste, généralement inférieurs à $10m^3$ afin de limiter les pertes dans le cas où le milieu réactionnel polymériserait.

La présente invention a pour objet un procédé de préparation de solution aqueuse de sels d'ammoniums quaternaires insaturés répondant à la formule générale (I) indiquée ci-dessus, et notamment des solutions aqueuses concentrées de ces sels (I) qui ne présente pas les problèmes de viscosité ni d'hydrolyse du monomère (II) décrit auparavant, et qui permet d'éliminer les risques de polymérisation du milieu réactionnel lors de la synthèse ainsi que ceux des solutions aqueuses du sel quaternaire (I) obtenues. Les solutions obtenues possèdent une stabilité à température ambiante supérieure à un an.

Plus précisément, l'invention a pour objet un procédé de préparation de solution aqueuse de sels d'ammonium quaternaire insaturé répondant à la formule (1) à partir d'au moins un monomère (méth)-acrylique (II) et d'au moins un agent quaternisant (III), en présence d'au moins un inhibiteur de polymérisation caractérisé en ce que :

- a) on fait réagir dans un réacteur fermé, le monomère (méth)acrylique (II) avec 5 à 20% de la quantité pondérale nécessaire à la réaction d'agent quaternisant (III), ce dernier étant introduit en continu dans le réacteur,
- b) ensuite, on ajoute en continu l'eau et le reste d'agent quaternisant (III) jusqu'à l'obtention de la concentration souhaitée de sel d'ammonium quaternaire (I) dans l'eau,
- c) on maintient la température pendant les étapes (a) et (b) à une valeur comprise entre 30 et 60 °C,
- d) et pendant les étapes (a) et (b) et à l'approche de la fin de la réaction en particulier, on maintient dans le milieu réactionnel un courant de gaz oxygéné tel que le rapport en volume de gaz total à la sortie du réacteur sur l'oxygène introduit à l'entrée de ce même réacteur est inférieur à 100/1.

De préférence, on impose lors de la réaction un rapport en volume de gaz sortant du réacteur sur l'oxygène introduit à l'entrée de ce réacteur inférieur à 50/1.

Conformément à une mise en oeuvre différente du procédé selon l'invention,

- a′) on introduit dans un réacteur fermé, le monomère (méth)acrylique (II) et 5 à 20% en poids d'une solution aqueuse de sel quaternaire (I) par rapport au poids des monomères (méth)acryliques (II) comprenant de 50 à 85% en poids de sel quaternaire, les conditions opératoires b, c et d restant, quant à elles, identiques à celles préconisées dans l'invention ci-dessus.

Dans le but d'éviter la sursaturation du milieu réactionnel en sel quaternaire (I), on impose pendant l'étape (b) un rapport molaire d'introduction d'eau sur l'agent quaternisant (III) compris entre 2,2 et 3,7. En dessous de 2,2, on obtient un milieu réactionnel visqueux dont on contrôle difficilement, voire pas du tout, la température. Par contre, à une valeur supérieure de 3,7, les risques d'hydrolyse du monomère (méth)-acrylique (II) sont importants.

De préférence, on maintient pendant les étapes (a), (a′) et (b) une température comprise entre 45 °C et 55 °C.

De préférence encore, pendant l'étape (a) ou (a′) indiquées précédemment on introduit environ 10% en poids respectivement d'agent quaternisant (III) ou d'une solution aqueuse de sel quaternaire (I) comprenant 50 à 85% en poids de sel quaternaire. C'est en effet dans cette mise en oeuvre préférée que l'on obtient la meilleure cinétique réactionnelle et la viscosité la plus satisfaisante.

Le procédé selon l'invention est de préférence conduit à une pression comprise entre la pression atmosphérique et 1,6 bars en pression absolue. En effet, à des valeurs de pression supérieure, la solution de sel quaternaire (I) n'est pas stable au stockage et parfois même polymérise en fin d'opération.

Lorsque l'agent quaternisant (III) est un composé volatil, gazeux à la température de réaction, l'introduction de l'agent quaternisant en cours de réaction est conduite de manière à limiter ses pertes dans les évents gazeux. Les pertes sont ainsi maintenues inférieures à 10% (molaire) de la stoeckiométrie.

Le gaz en sortie du réacteur est ensuite conduit vers un dispositif de traitement visant à le débarrasser des traces de l'agent quaternisant (III) qu'il contient, en particulier dans le cas où ce dernier possède une tension de vapeur élevée ou est volatil à la température de réaction. De préférence, on envoie le gaz dans un second réacteur comprenant du monomère (méth)acrylique (II) dans lequel l'agent quaternisant (III) est piégé.

En fin de réaction, les traces d'agent quaternisant (III) dissous dans le mélange réactionnel sont éliminées par un balayage de ce dernier avec un fort débit de gaz oxygéné, par exemple de l'air.

Le procédé selon l'invention est utilisé à la quaternisation des monomères (méth)acryliques (II) (se reporter à la formule indiquée auparavant). Toutefois, ce procédé est plus particulièrement destiné aux monomères (méth)acryliques (II) facilement hydrolysables et susceptibles de polymériser facilement. Parmi ces derniers, on peut citer l'acrylate de diméthylaminoéthyle, l'acrylate de diméthylaminopropyle, le méthacrylate de diméthylaminoéthyle, le méthacrylate de diméthylaminopropyle, le diméthylaminopropyle acrylamide, le diméthylaminopropyle méthacrylamide.

Les agents quaternisants (III) convenant bien à la présente invention sont notamment les hydrocarbures halogénés tels que le chlorure de méthyle, le bromure de méthyle, l'iodure de méthyle, le chlorure d'éthyle,

le bromure d'éthyle, l'iodure d'éthyle, le chlorure de benzyle; le sulfate de diméthyle ou le carbonate de diméthyle.

Parmi les stabilisants (ou inhibiteurs de polymérisation) utilisables dans le procédé selon l'invention, on peut citer le toluène -3,5-diterbutyl-4-hydroxy, l'éther méthylique d'hydroquinone, la phénothiazine, l'hydroquinone, le catéchol et le terbutyl catéchol ou encore un mélange de ces stabilisants. De préférence, on utilise de 100 à 1500 ppm de stabilisant par rapport au monomère (méth)acrylique (II). Les recherches effectuées à ce sujet par la demanderesse ont montré qu'on obtient de bons résultats en utilisant de 1000 à 1500 ppm de toluène -3,5-diterbutyl-4-hydroxy, ou 500 à 1000 ppm d'étherméthylique d'hydroquinone, ou 70 ppm de phénothiazine, ces quantités étant exprimées par rapport à la quantité de monomères (méth)-acryliques (II).

Le procédé selon l'invention permet de préparer des solutions aqueuses de sels quaternaires (I), notamment à des concentrations de 50 à 85% en poids de sels (I) dans l'eau. Les solutions de sels quaternaires (I) ainsi obtenues contiennent des quantités faibles de produits d'hydrolyse des monomères (II) et donc ne nécessitent pas de purification complémentaire. Ces solutions possèdent une stabilité au stockage à température ambiante supérieure à un an.

De plus, en raison de la bonne reproductibilité du procédé selon l'invention, en particulier de la stabilité du mélange réactionnel, on effectue des opérations de quaternisation dans des réacteurs de grande dimension, de l'ordre de 20 à 50 $m^3$.

Les exemples qui vont suivre, donnés à titre indicatif, permettront de mieux comprendre l'invention. Dans ces exemples, les pourcentages indiqués sont des % en poids.

**EXEMPLE 1 (comparatif)**

**Préparation d'une solution aqueuse de chlorure de méthacryloyloxyéthyltriméthylammonium à 75%**

Dans un réacteur à double enveloppe, sous agitation, on charge 360 kg de méthacrylate de diméthylaminoéthyle comprenant 1000 ppm d'étherméthylique d'hydroquinone.

Après pressurisation du réacteur à 1,6 bar absolu d'air, on introduit en continu du chlorure de méthyle et de l'eau dans un rapport pondéral eau/$CH_3Cl$ égal à 1,3 (soit un rapport molaire égal à 3,6). La réaction est conduite à 40 °C et à une pression qui évolue progressivement jusqu'à 5 bars absolus en fin de réaction.

Dans cet exemple, la quaternisation est menée sans sortie d'air ou de chlorure de méthyle et sans apport d'air.

Lors de la remise du réacteur à la pression atmosphérique en fin de réaction, il se produit une polymérisation instantanée du mélange réactionnel. On pense que cette polymérisation est en partie provoquée par la vaporisation du chlorure de méthyle excédentaire dissous dans le mélange réactionnel, qui à cette occasion entraine avec lui l'oxygène dissous, produisant ainsi un défaut d'oxygène dans le mélange réactionnel.

**EXEMPLE 2 (comparatif)**

**Préparation d'une solution aqueuse de chlorure de méthacryloyloxyéthyltriméthylammonium à 75%**

Dans un réacteur, sous agitation, on charge 360 kg de méthacrylate de diméthylaminoéthyle comportant 1000 ppm d'étherméthylique d'hydroquinone. Pendant toute la durée de la réaction on maintient une pression à 3 bars absolus dans le réacteur avec une introduction en continu de 100 Normolitres/heure (Nl/h) d'air. La pression du réacteur est régulée par la sortie des évents qui contiennent du chlorure de méthyle non transformé et de l'air.

Dans une première étape, on introduit en continu dans le réacteur du chlorure de méthyle à raison de 10 kg/h. Après 1,5 heure on introduit en continu l'eau et le chlorure de méthyle dans un rapport pondéral eau/chlorure de méthyle égal à 1,2 (soit un rapport molaire de 3,3).

La température de réaction est maintenue à 40 °C.

En fin de réaction, le réacteur est refroidi à 20 °C, puis, simultanément à l'introduction de 10 $Nm^3/h$ d'air dans la phase liquide, il est remis à la pression atmosphérique. Le chlorure de méthyle excédentaire dissous en quantité importante dans la masse réactionnelle est alors instantanément vaporisé. Ensuite on injecte pendant 2 heures dans le réacteur de l'air à raison de 10 $Nm^3/h$ afin de chasser les traces de

chlorure de méthyle et de réoxygéner le produit.

La solution aqueuse de sel quaternaire ainsi obtenue, stockée à température ambiante et à l'abri de la lumière polymérise après 15 jours de stockage.

**EXEMPLE 3 :**

**Préparation d'une solution aqueuse de chlorure de méthacryloyloxyéthyltriméthylammonium à 75%**

Dans un réacteur à double enveloppe, sous agitation, on charge 360 kg de méthacrylate de diméthylaminoéthyle stabilisé avec 1500 ppm de toluène-3,5-diterbutyl-4-hydroxy.

Pendant toute la durée de la réaction, soit au total 10 heures, on maintient la température à 50 °C, la pression atmosphérique et un débit d'air continu de 120 Nl/h à l'entrée du réacteur et un débit des évents à la sortie du réacteur inférieur ou égal à 1,2 $Nm^3/h$, c'est-à-dire un rapport en volume des évents sur l'oxygène introduit dans le réacteur inférieur ou égal à 50.

On introduit le chlorure de méthyle à un débit de 13 kg/h pendant une heure, soit 11,2 % de la quantité totale de $CH_3Cl$ nécessaire à la réaction. Puis on introduit simultanément l'eau et le chlorure de méthyle à des débits respectifs de 20 kg/h et de 16 kg/h, soit un rapport molaire eau/$CH_3Cl$ égal à 3,5.

A l'approche de la fin de la réaction, c'est-à-dire après environ 7 heures, le débit de chlorure de méthyle est progressivement réduit jusqu'à 3 kg/h.

Ensuite, on injecte dans le produit fabriqué de l'air à raison de 10 $Nm^3/h$ pendant 2 heures.

Le chlorure de méthacryloyloxyéthyltriméthylammonium à 75% ainsi fabriqué contient 0,13% d'acide méthacrylique et 0,75% de méthacrylate de diméthylaminoéthyle.

Stocké à température ambiante et à l'abri de la lumière, ce produit présente une stabilité supérieure à 1 an.

**EXEMPLE 4 (comparatif)**

**Préparation d'une solution aqueuse de chlorure de méthacryloyloxyéthyltriméthylammonium à 75%**

Dans un réacteur chargé de 360 kg de méthacrylate de diméthylaminoéthyle stabilisé avec 1500 ppm de toluène-3,5-diterbutyl-4-hydroxy, on introduit en continu 30 Nl/h d'air pendant toute l'opération, et on maintient la température à 50 °C, la pression atmosphérique et un débit de sortie des évents d'au plus 1 $Nm^3/h$ pendant toute sa durée. Le rapport en volume des évents sur l'oxygène à l'entrée du réacteur est alors de 166.

On introduit pendant 1 heure 13 kg de chlorure de méthyle, soit 11,2 % de la quantité totale de $CH_3Cl$ nécessaire à la réaction. Ensuite, on injecte simultanément de l'eau et du chlorure de méthyle à des débits respectifs de 20 kg/h et 16 kg/h, soit un rapport molaire eau/$CH_3Cl$ égal à 3,5.

A l'approche de la fin de réaction, le débit de chlorure de méthyle est réduit à 10 kg/h, et le débit de sortie des évents monte jusqu'à 4,5 $Nm^3/h$ pendant une période de l'ordre de 15 minutes à 1 heure. Le rapport en volume des évents sur l'oxygène à l'entrée du réacteur est alors de 750. Après l'arrêt de l'introduction de chlorure de méthyle, on injecte dans le produit fabriqué de l'air à raison de 10 $Nm^3/h$ pendant 2 heures.

Le produit final, stocké à l'abri de la lumière et à température ambiante présente une tenue à la polymérisation variable dans le temps, de 4 semaines à plusieurs mois.

**EXEMPLES 5 à 7**

**Préparation d'une solution aqueuse de chlorure de méthacryloyloxyéthyltriméthylammonium à 75%**

Influence de la concentration de sel quaternaire présent dans le première étape a) du procédé selon l'invention.

En dehors des indications données dans le tableau ci-dessous, les conditions opératoires sont identiques à celles utilisées dans l'exemple 3.

Dans le tableau en annexe, on désignera par :

(I) : le chlorure de méthacryloyloxyéthyltriméthylammonium

(II) : le méthacrylate de diméthylaminoéthyle
(III) : le chlorure de méthyle
(IV) : l'acide méthacrylique

* Dans les essais 6 et 7, on constate après 1 heure d'introduction d'eau (étape b), une montée incontrôlable de la température du milieu réactionnel dans le réacteur de 5 à 10 °C voire supérieure. Ce phénomène est lié à la sursaturation du milieu réactionnel en sel quaternaire (I) limitant ainsi les échanges thermiques avec le fluide caloporteur circulant dans la double enveloppe du réacteur. En conséquence, les conditions opératoires des essais 6 et 7 (se reporter au tableau en annexe) ne peuvent pas être maintenues jusqu'à la fin de la réaction. Une dilution du milieu réactionnel avec de l'eau et l'arrêt de l'injection de $CH_3Cl$ sont nécessaires au cours de la réaction (étape b) de manière à se rapprocher des conditions opératoires de l'essai n° 5. En effet, on bénéficie dans l'essai n°5 de conditions stables de fonctionnement du réacteur pendant la réaction. De plus, le produit obtenu possède une excellente stabilité au stockage.

## EXEMPLE 8

### Préparation d'une solution aqueuse de chlorure de méthacryloyloxyéthyltriméthylammonium à 80%

Dans un réacteur industriel d'une capacité de 8 $m^3$, à double enveloppe et muni d'un dispositif d'agitation en fonctionnement, on charge 5220 kg de méthacrylate de diméthylaminoéthyle stabilisé à l'aide de 1500 ppm de toluène-3,5-diterbutyl-4-hydroxy et de 160 ppm d'éthermêthylique d'hydroquinone. On injecte en continu dans le réacteur 2000 Nl/h d'air pendant toute la durée de la réaction. De plus, pendant la réaction, on maintient la température à 50 °C, la pression atmosphérique, et un débit de sortie des évents (chlorure de méthyle + air) entre 2000 et 7000 Nl/h. Le rapport en volume des évents sur l'oxygène introduit dans le réacteur varie donc entre 5 et 17,5.

Pendant la première étape de la réaction, on injecte dans le réacteur 170 kg de chlorure de méthyle en continu à un débit de 100-110 kg/h, soit 10% de la quantité totale de chlorure de méthyle nécessaire à la réaction.

Puis, on introduit simultanément en continu du chlorure de méthyle et de l'eau dans un rapport pondéral eau/$CH_3Cl$ égal à 1,2 (soit un rapport molaire eau/$CH_3Cl$ égal à 3,3).

A l'approche de la fin de la réaction, après 12 heures le débit de chlorure de méthyle est progressivement réduit à 35 kg/h tandis que le débit de sortie des évents est maintenu inférieur à 12000 Nl/h. Le rapport en volume des évents sur l'oxygène introduit dans le réacteur est alors de 30.

L'opération est arrêtée après 14h de réaction environ.

Pour cette opération on a utilisé 1720 kg d'eau, 1770 kg de chlorure de méthyle et on a obtenu 8500 kg de chlorure de méthacryloyloxyéthyltriméthylammonium à 80% dans l'eau.

Le produit obtenu est ensuite soumis à une insufflation d'air avec un débit de 70 $Nm^3$/h pendant 1,5 h à 45-50 °C, puis pendant 2 heures à température ambiante afin d'éliminer le chlorure de méthyle dissous. Le rapport en volume des évents sur l'oxygène introduit dans le réacteur est alors de 5.

Le produit final obtenu a les caractéristiques suivantes : (quantités données en %)
eau : 20
acide méthacrylique : 0,13
méthacrylate de diméthylaminoéthyle : 0,14
chlorure de méthyle : 40 ppm
polymère : néant

Le produit ainsi fabriqué présente une stabilité au stockage supérieure à 1 an.

## EXEMPLE 9

### Préparation d'une solution aqueuse de chlorure d'acryloyloxyéthyltriméthylammonium à 80%

Dans un réacteur industriel d'une capacité de 8 $m^3$ agité et à double enveloppe, on charge 5150 kg d'acrylate de diméthylaminoéthyle stabilisé à l'aide de 700 ppm d'éther méthylique d'hydroquinone.

Pendant toute la durée de la réaction, on injecte en continu dans le réacteur 2000 Nl/h d'air et on maintient :

- la température à 50 °C,
- la pression atmosphérique,
- le débit de sortie des évents inférieur à 7000 Nl/h (soit un rapport en volume des évents sur l'oxygène

introduit dans le réacteur inférieur à 17,5).

Dans la première étape, on injecte dans le réacteur 180 kg de chlorure de méthyle avec un débit de 110 kg/h, (soit 11% de la quantité totale de $CH_3Cl$ nécessaire à la réaction), puis dans une seconde étape, on injecte simultanément et en continu le chlorure de méthyle et l'eau dans un rapport pondéral eau/$CH_3Cl$ compris entre 0,9 et 1,0 (d'où un rapport molaire eau/$CH_3Cl$ compris entre 2,5 et 2,8).

A l'approche de la fin de la réaction, le débit de chlorure de méthyle est progressivement réduit à 35 kg/h et le débit de sortie des évents est maintenu inférieur à 12000 Nl/h (d'où un rapport en volume des évents sur l'oxygène à l'entrée du réacteur inférieur à 30).

L'opération est arrêtée après 15 heures de réaction.

Dans cette opération, on a utilisé 1 749 kg d'eau et 1 961 kg de chlorure de méthyle et on récupère 8 620 kg de chlorure d'acryloyloxyéthyltriméthylammonium à 80 % dans l'eau.

Le produit final est ensuite soumis à une injection d'air avec un débit de 70 $Nm^3$/l pendant 1,5 h à chaud puis pendant 1,5 h à température ambiante.

Le produit final obtenu a les caractéristiques suivantes : (quantités données en %)

eau : 20,4
acide acrylique : 0,69
acrylate de diméthylaminoéthyle : 1,3
chlorure de méthyle : 15 ppm
polymère : néant

Ce produit, stocké à température ambiante et à l'abri de la lumière, présente une stabilité supérieure à 1 an.

## EXEMPLE 10

### Préparation d'une solution aqueuse de chlorure de méthacryloyloxyéthyle benzyl diméthylammonium à 80%

Dans un réacteur double enveloppe, sous agitation, on charge 250 kg de méthacrylate de diméthylaminoéthyle stabilisé à 1500 ppm de BHT.

Pendant toute la durée de la réaction, soit au total 6 heures, on maintient la température à 50° C et un débit d'air continu de 120 Nl/h.

On introduit le chlorure de benzyle à un débit de 30 kg/h pendant 1 heure, soit 14,9% de la quantité totale nécessaire. Puis on introduit simultanément l'eau et le chlorure de benzyle à des débits respectifs de 23 kg/h et 46 kg/h soit un rapport molaire eau/chlorure de benzyle égal à 3,5.

On introduit au total 201 kg de chlorure de benzyle et 113 kg d'eau.

Le sel d'ammonium à 80% ainsi fabriqué contient 0,3% d'acide méthacrylique et 0,3% de méthacrylate de diméthylaminoéthyle.

Ce produit est stable pendant plus d'un an au stockage.

## EXEMPLE 11

### Préparation d'une solution aqueuse de chlorure d'acryloyloxyéthyltriméthylammonium à 80%

Dans un réacteur industriel de $8m^3$ à double enveloppe, on charge initialement 4 500 kg d'acrylate de diméthylaminoéthyle stabilité à 700 ppm d'étherméthylique d'hydroquinone et 1 100 kg d'une solution aqueuse à 80 % de chlorure d'acryloyloxyéthyltriméthylammonium.

Puis on introduit dans le réacteur simultanément et en continu le chlorure de méthyle et l'eau à des rapports de débit pondéraux compris entre 1,0/1,0 et 0,9/1,0.

La réaction est conduite à pression atmosphérique, à une température de 52° C et avec introduction en continu de 2 000 Nl/h d'air dans le réacteur. Le débit des gaz résiduaires est maintenu inférieur à 7 000 Nl/h.

A l'approche de la fin de réaction, le débit de chlorure de méthyle est progressivement réduit jusqu'à 20 kg/h et le débit de sortie des évents est maintenu inférieur à 12 000 Nl/h. Le rapport en volume des évents sur l'oxygène introduit dans le réacteur est alors de 30.

La réaction est finie après 13 heures d'introduction de chlorure de méthyle.

Le produit fini, après injection d'air pendant 3 heures à un débit de 60 $Nm^3$/h, a les caractéristiques suivantes :

eau : 20,10 %

acide acrylique : 0,85 %
acrylate de diméthylaminoéthyle : 1,20 %
chlorure de méthyle : 10 ppm
polymère : néant

Le produit offre une stabilité au stockage supérieure à 1 an.

Dans cette opération on a utilisé au total :
4500 kg d'Adame
1100 kg d'Adquat MC 80
1685 kg de chlorure de méthyle
1522 kg d'eau
et on récupère 8625 kg d'Adquat MC 80.

## ANNEXE : TABLEAU

| N° de l'essai | Etape a (durée : 1 h) | | Etape b | | Impuretés présentes dans le propduit final | |
|---|---|---|---|---|---|---|
| | Quantités de réactifs introduits (kg) | Composition du mélange obtenue (%) | (III) introduit (kg/h) | eau introduite (kg/h) | teneur en (II) (%) | teneur en (IV) (%) |
| **5** | (II) : 360<br>(III) : 10 | (I) : 10<br>(II) : 90 | 20 | 20 | 0,15 | 0,13 |
| **6*** | (II) : 360<br>(III) : 30 | (I) : 30<br>(II) : 70 | 20 | 30 | 0,15 | 0,12 |
| **7*** | (II) : 360<br>(III) : 40 | (I) : 40<br>(II) : 60 | 20 | 40 | 0,15 | 0,12 |

## Revendications

**1.** Procédé de préparation de solution aqueuse de sels d'ammonium quaternaire insaturés répondant à la formule (1) suivante :

$$H_2C = C(R_3) - C(O) - A - R_4 - \overset{\oplus}{N}(R_1)(R_2)(R),X^{\ominus}$$

dans laquelle :

- A est un atome d'oxygène ou un groupe NH,
- $R_3$ est un atome d'hydrogène ou un radical méthyle,
- $R_4$ est un radical alkyle, linéaire ou ramifié, de 2 à 4 atomes de carbone,
- $R_1$ et $R_2$, différents ou identiques, est un radical alkyle ou un radical aryle,
- R est un radical alkyle ou aryle,
- X est choisi parmi Cl, Br, I, $CH_3$-$CO_3$ ou $CH_3$-$SO_4$,

à partir d'au moins un monomère (méth)acrylique (II) de formule :

$$H_2C = C(R_3) - C(O) - A - R_4 - N(R_1)(R_2)$$

et d'au moins un agent quaternisant (III) de formule RX, en présence d'au moins un inhibiteur de polymérisation, procédé caractérisé en ce que :

- a) on fait réagir dans un réacteur fermé, le monomère (méth)acrylique (II) avec 5 à 20% de la quantité pondérale nécessaire à la réaction d'agent quaternisant (III), ce dernier étant introduit en continu dans le réacteur,
- b) ensuite, on ajoute en continu l'eau et le reste d'agent quaternisant (III) jusqu'à l'obtention de la concentration souhaitée de sel d'ammonium quaternaire (I) dans l'eau, le rapport molaire eau sur agent quanternisant (III) étant compris entre 2,2 et 3,7.
- c) on maintient la température pendant les etapes (a) et (b) à une valeur comprise entre 30 et 60 °C,
- d) et pendant les étapes (a) et (b) et à l'approche de la fin de la réaction en particulier, on maintient dans le milieu réactionnel un courant de gaz oxygéné tel que le rapport en volume de gaz total à la sortie du réacteur sur l'oxygène introduit à l'entrée de ce même réacteur est inférieur à 100/1.

**2.** Procédé selon la revendication 1, caractérisé en ce que la température pendant les étapes (a) et (b) est comprise entre 45 °C et 55 °C.

**3.** Procédé selon la revendication 1 ou 2, caractérisé en ce que pendant l'étape (a) on introduit 10% en poids d'agent quaternisant.

**4.** Procédé selon la revendication 1, 2 ou 3, caractérisé en ce que la réaction est effectuée à une pression absolue comprise entre la pression atmosphérique et 1,6 bars.

**5.** Procédé selon l'une quelconque des revendications 1 à 4, caractérisé en ce que le gaz en sortie du réacteur est conduit vers un dispositif de traitement visant à le débarasser des traces d'agent quaternisant RX qui est constitué d'un second réacteur de quaternisation comprenant du monomère (méth)acrylique.

**6.** Procédé selon l'une quelconque des revendications 1 à 5, caractérisé en ce que l'on utilise de 100 à 1500 ppm d'inhibiteur de polymérisation par rapport au monomère (méth)acrylique.

**7.** Procédé de préparation de solution aqueuse de sels d'ammonium quaternaires insaturés répondant à la formule (1) suivante :

$$H_2C = C(R_3) - C(O) - A - R_4 - \overset{\oplus}{N}(R_1)(R_2)(R),X^{\ominus}$$

dans laquelle :

- A est un atome d'oxygène ou un groupe NH,
- $R_3$ est un atome d'hydrogène ou un radical méthyle,
- $R_4$ est un radical alkyle, linéaire ou ramifié, de 2 à 4 atomes de carbone,
- $R_1$ et $R_2$, différents ou identiques, est un radical alkyle ou un radical aryle,

- R est un radical alkyle ou aryle,
- X est choisi parmi Cl, Br, I, $CH_3$-$CO_3$ ou $CH_3$-$SO_4$,

à partir d'au moins un monomère (méth)acrylique (II) de formule :

$H_2C = C(R_3) - C(O) - A - R_4 - N(R_1)(R_2)$

et d'au moins un agent quaternisant (III) de formule RX, en présence d'au moins un inhibiteur de polymérisation, procédé caractérisé en ce que :

- a') on introduit, dans un réacteur fermé, le monomère (méth)acrylique (II) et 5 à 20% en poids d'une solution aqueuse de sel quaternaire (I) par rapport au poids de monomères (méth)acryliques (II) comprenant 50 à 85% en poids de sel quaternaire,
- b) ensuite, on ajoute en continu l'eau et le reste d'agent quaternisant (III) jusqu'à l'obtention de la concentration souhaitée de sel d'ammonium quaternaire (I) dans l'eau, le rapport molaire eau sur agent quarternisant (III) étant compris entre 2,2 et 3,7.
- c) on maintient la température pendant les étapes (a') et (b) à une valeur comprise entre 30 et 60 °C,
- d) et pendant les étapes (a') et (b) et à l'approche de la fin de la réaction en particulier, on maintient dans le milieu réactionnel un courant de gaz oxygéné tel que le rapport en volume de gaz à la sortie du réacteur sur l'oxygène introduit à l'entrée de ce même réacteur est inférieur à 100/1.

**8.** Procédé selon la revendication 7, caractérisé en ce que la température pendant les étapes (a′) et (b) est comprise entre 45 °C et 55 °C

**9.** Procédé selon la revendication 7 ou 8, caractérisé en ce que pendant l'étape (a′), on introduit 10% en poids d'une solution aqueuse de sel quaternaire comprenant 50 à 80% en poids de sel quaternaire.

**10.** Procédé selon la revendication 7, 8 ou 9, caractérisé en ce que la réaction est effectuée à une pression absolue comprise entre la pression atmosphérique et 1,6 bars.

**11.** Procédé selon l'une quelconque des revendications 7 à 10, caractérisé en ce que le gaz en sortie du réacteur est conduit vers un dispositif de traitement visant à le débarasser des traces d'agent quaternisant RX qui est constitué d'un second réacteur de quaternisation comprenant du monomère (méth)acrylique.

**12.** Procédé selon l'une quelconque des revendications 7 à 11, caractérisé en ce que l'on utilise de 100 à 1500 ppm d'inhibiteur de polymérisation par rapport au monomère (méth)acrylique.

**Claims**

**1.** Process for preparing an aqueous solution of unsaturated quaternary ammonium salts corresponding to the following formula (I):

$$H_2C = C(R_3) - C(O) - A - R_4 - {}^{\oplus}N(R_1)(R_2)(R), X^{\ominus}$$

in which

- A is an oxygen atom or an NH group,
- $R_3$ is a hydrogen atom or a methyl radical,
- $R_4$ is a linear or branched alkyl radical, having from 2 to 4 carbon atoms,
- $R_1$ and $R_2$, which may be different or identical, are an alkyl radical or an aryl radical,
- R is an alkyl or aryl radical,
- X is selected from Cl, Br, I, $CH_3$-$CO_3$ or $CH_3SO_4$,

from at least one (meth)acrylic monomer (II) of formula:

$H_2C = C(R_3) - C(O) - A - R_4 - N(R_1)(R_2)$

and at least one quaternising agent (III) of formula RX, in the presence of at least one polymerisation inhibitor, which process is characterised in that:

- a) the (meth)acrylic monomer (II) is reacted in a closed reactor with 5 to 20% of the weight quantity necessary for the reaction of the quaternising agent (III), the latter being introduced in continuous fashion into the reactor,
- b) then, water and the remainder of the quaternising agent (III) are added in continuous fashion until the desired concentration of quaternary ammonium salt (I) in the water is obtained, the molar ratio of water to quaternising agent (III) being between 2.2 and 3.7,
- c) the temperature is maintained during steps (a) and (b) at a value between 30 and 60 °C,
- d) and during steps (a) and (b) and at the approach to the end of the reaction in particular, a stream of oxygen-containing gas is maintained in the reaction medium such that the ratio by volume of total gas at the reactor exit to oxygen introduced at the entry of this same reactor is less than 100:1.

**2.** Process according to Claim 1, characterised in that the temperature during steps (a) and (b) is between 45 °C and 55 °C.

**3.** Process according to Claim 1 or 2, characterised in that, during step (a), 10% by weight of quaternising agent is introduced.

**4.** Process according to Claim 1, 2 or 3, characterised in that the reaction is performed at an absolute pressure between atmospheric pressure and 1.6 bars.

**5.** Process according to any one of Claims 1 to 4, characterised in that the gas leaving the reactor is led to a treatment device whose objective is to rid it of traces of quaternising agent RX, which consists of a second quaternisation reactor comprising (meth)acrylic monomer.

**6.** Process according to any one of Claims 1 to 5, characterised in that from 100 to 1500 ppm of polymerisation inhibitor is used relative to the (meth)acrylic monomer.

**7.** Process for preparing an aqueous solution of unsaturated quaternary ammonium salts corresponding to the following formula (I):

$$H_2C = C(R_3) - C(O) - A - R_4 - {}^{\oplus}N(R_1)(R_2)(R), X^{\ominus}$$

in which:

- A is an oxygen atom or an NH group,
- $R_3$ is a hydrogen atom or a methyl radical,
- $R_4$ is a linear or branched alkyl radical having 2 to 4 carbon atoms,
- $R_1$ and $R_2$, which may be different or identical, are an alkyl radical or an aryl radical,
- R is an alkyl or aryl radical,
- X is selected from Cl, Br, I, $CH_3$-$CO_3$ or $CH_3$-$SO_4$,

from at least one (meth)acrylic monomer (II) of formula:

$$H_2C = C(R_3) - C(O) - A - R_4 - N(R_1)(R_2)$$

and at least one quaternising agent (III) of formula RX, in the presence of at least one polymerisation inhibitor, which process is characterised in that:

- a') the (meth)acrylic monomer (II) and 5 to 20% by weight of an aqueous solution of quaternary salt (I) relative to the weight of (meth)acrylic monomers (II) comprising 50 to 85% by weight of quaternary salt are introduced into a closed reactor,
- b) then, water and the remainder of the quaternising agent (III) are added in continuous fashion until the desired concentration of quaternary ammonium salt (I) in the water is obtained, the molar ratio of water to quaternising agent (III) being between 2.2 and 3.7,
- c) the temperature is maintained during steps (a') and (b) at a value between 30 and 60 °C,

- d) and during steps (a') and (b) and at the approach to the end of the reaction in particular, a stream of oxygen-containing gas is maintained in the reaction medium such that the ratio by volume of gas at the reactor exit to oxygen introduced at the entry of this same reactor is less than 100:1.

**8.** Process according to Claim 7, characterised in that the temperature during steps (a') and (b) is between 45° C and 55° C.

**9.** Process according to Claim 7 or 8, characterised in that, during step (a'), 10% by weight of an aqueous solution of quaternary salt comprising 50 to 80% by weight of quaternary salt is introduced.

**10.** Process according to Claim 7, 8 or 9, characterised in that the reaction is performed at an absolute pressure between atmospheric pressure and 1.6 bars.

**11.** Process according to any one of Claims 7 to 10, characterised in that the gas at outflow from the reactor is led to a treatment device whose objective is to rid it of traces of quaternising agent RX, which consists of a second quaternisation reactor comprising (meth)acrylic monomer.

**12.** Process according to any one of Claims 7 to 11, characterised in that from 100 to 1500 ppm of polymerisation inhibitor is used relative to the (meth)acrylic monomer.

**Patentansprüche**

**1.** Verfahren zur Herstellung einer wäßrigen Lösung von ungesättigten quartären Ammoniumsalzen entsprechend der nachstehenden Formel (I):

$$H_2C = C(R_3) - C(O) - A - R_4 - \overset{\oplus}{N}(R_1)(R_2)(R),X^{\ominus}$$

wären:

- A ein Sauerstoffatom oder eine NH-Gruppe ist,
- $R_3$ ein Wasserstoffatom oder ein Methylrest ist,
- $R_4$ ein linearer oder verzweigter Alkylrest mit 2 bis 4 Kohlenstoffatomen ist.
- $R_1$ und $R_2$, verschieden oder identisch, ein Alkylrest oder ein Arylrest sind,
- R ein Alkyl- oder Arylrest ist,
- X ausgewählt ist aus der Gruppe bestehend aus Cl, Br, J, $CH_3$ - $CO_3$ und $CH_3$ - $SO_4$,

ausgehend von mindestens einem (Meth)acryl-Monomer (II) der Formel:

$H_2C = C(R_3)\text{-}C(O)\text{-}A\text{-}R_4\text{-}N(R_1)(R_2)$

und mindestens einem Quaternisierungsmittel (III) der Formel RX, in Gegenwart mindestens eines Polymerisationsinhibitors, wobei das Verfahren dadurch gekennzeichnet ist, daß

- a) das (Meth)acryl-Monomer (II) mit 5 bis 20 % der für die Reaktion erforderlichen Gewichtsmenge an Quaternisierungsmittel (III) in einem geschlossenen Reaktor reagieren gelassen wird, wobei das Quaternisierungsmittel kontinuierlich in den Reaktor eingebracht wird,
- b) sodann kontinuierlich das Wasser und der Rest des Quaternisierungsmittels (III) bis zum Erhalt der gewünschten Konzentration des quartären Ammoniumsalzes (I) in dem Wasser zugegeben werden, wobei das Molverhältnis Wasser zu Quaternisierungsmittel (III) zwischen 2,2 und 3,7 beträgt,
- c) die Temperatur während der Schritte (a) und (b) auf einem Wert zwischen 30° und 60° C gehalten wird, und
- d) während der Schritte (a) und (b) und insbesondere bei Annäherung an das Ende der Reaktion in dem Reaktionsmilieu ein solcher sauerstoffhaltiger Gasstrom aufrechterhalten wird, daß das Volumenverhältnis des gesamten Gases am Ausgang des Reaktorszu dem am Eingang dieses Reaktorseingebrachten Sauerstoff weniger als 100/1 beträgt.

**2.** Verfahren nach Anspruch 1, dadurch gekennzeichnet, daß die Temperatur während der Schritte (a) und (b) zwischen 45° und 55° C beträgt.

**3.** Verfahren nach Anspruch 1 oder 2, dadurch gekennzeichnet, daß während des Schritts (a) 10 Gew.-% des Quaternisierungsmittels eingebracht werden.

**4.** Verfahren nach Anspruch 1, 2 oder 3, dadurch gekennzeichnet, daß die Reaktion bei einem zwischen dem Atmosphärendruck und 1,6 bar liegenden Absolutdruck durchgeführt wird.

**5.** Verfahren nach irgendeinem der Ansprüche 1 bis 4, dadurch gekennzeichnet, daß das aus dem Reaktor austretende Gas zu einer Behandlungsvorrichtung geleitet wird, die darauf gerichtet ist, es von Spuren des Quaternisierungsmittels RX zu befreien, und die von einem (Meth)acryl-monomer enthaltenden zweiten Quaternisierungsreaktor gebildet ist.

**6.** Verfahren nach irgendeinem der Ansprüche 1 bis 5, dadurch gekennzeichnet, daß 100 bis 1500 ppm Polymerisationsinhibitor, bezogen auf das (Meth)acryl-Monomer, eingesetzt werden.

**7.** Verfahren zur Herstellung einer wäßrigen Lösung von ungesättigten quartären Ammoniumsalzen entsprechend der nachstehenden Formel (I):

$$H_2C = C(R_3) - C(O) - A - R_4 - \overset{\oplus}{N}(R_1)(R_2)(R),X^{\ominus}$$

wären:

- A ein Sauerstoffatom oder eine NH-Gruppe ist,
- $R_3$ ein Wasserstoffatom oder ein Methylrest ist,
- $R_4$ ein linearer oder verzweigter Alkylrest mit 2 bis 4 Kohlenstoffatomen ist,
- $R_1$ und $R_2$, verschieden oder identisch, ein Alkylrest oder ein Arylrest sind,
- R ein Alkyl- oder Arylrest ist,
- X ausgewählt ist aus der Gruppe bestehend aus Cl, Br, J, $CH_3$ - $CO_3$ und $CH_3$-$SO_4$,

ausgehend von mindestens einem (Meth)acryl-Monomer (II) der Formel:

$H_2C = C(R_3)$-C(O)-A-$R_4$-N($R_1$)($R_2$)

und mindestens einem Quaternisierungsmittel (III) der Formel RX, in Gegenwart mindestens eines Polymerisationsinhibitors, wobei das Verfahren dadurch gekennzeichnet ist, daß

- a) das (Meth)acryl-Monomer (II) und, bezogen auf das Gewicht der (Meth)acryl-Monomere (II), 5 bis 20 Gew.-% einer wäßrigen Lösung quartären Salzes (I), die 50 bis 85 Gew.-% quartäres Salz enthält, in einen geschlossenen Reaktor ein gebracht werden,
- b) sodann kontinuierlich das Wasser und der Rest des Quaternisierungsmittels (III) bis zum Erhalt der gewünschten Konzentration des quartären Ammoniumsalzes (I) in dem Wasser zugegeben werden, wobei das Molverhältnis Wasser zu Quaternisierungsmittel (III) zwischen 2,2 und 3,7 beträgt,
- c) die Temperatur während der Schritte (a') und (b) auf einem Wert zwischen 30° und 60° C gehalten wird, und
- d) während der Schritte (a') und (b) und insbesondere bei Annäherung an das Ende der Reaktion in dem Reaktionsmilieu ein solcher sauerstoffhaltiger Gasstrom aufrechterhalten wird, daß das Volumenverhältnis des Gases am Ausgang des Reaktors zu dem am Eingang dieses Reaktors eingebrachten Sauerstoff weniger als 100/1 beträgt.

**8.** Verfahren nach Anspruch 7, dadurch gekennzeichnet, daß die Temperatur während der Schritte (a') und (b) zwischen 45° und 55° C beträgt.

**9.** Verfahren nach Anspruch 7 oder 8, dadurch gekennzeichnet, daß während des Schritts (a') 10 Gew.-% einer wäßrigen Lösung quartären Salzes eingebracht werden, die 50 bis 80 Gew.-% quartäres Salz enthält.

**10.** Verfahren nach Anspruch 7, 8 oder 9, dadurch gekennzeichnet, daß die Reaktion bei einem zwischen dem Atmosphärendruck und 1,6 bar liegenden Absolutdruck durchgeführt wird.

**11.** Verfahren nach irgendeinem der Ansprüche 7 bis 10, dadurch gekennzeichnet, daß das aus dem

Reaktor austretende Gas zu einer Behandlungsvorrichtung geleitet wird, die darauf gerichtet ist, es von Spuren des Quaternisierungsmittels RX zu befreien, und die von einem (Meth)acryl-Monomer enthaltenden zweiten Quaternisierungsreaktor gebildet ist.

**12.** Verfahren nach irgendeinem der Ansprüche 7 bis 11, dadurch gekennzeichnet, daß 100 bis 1500 ppm Polymerisationsinhibitor, bezogen auf das (Meth)acryl-Monomer, eingesetzt werden.